Europäisches Patentamt

European Patent Office

Office européen des brevets

(11) Veröffentlichungsnummer: **0 003 567**
**B1**

(12) **EUROPÄISCHE PATENTSCHRIFT**

(45) Veröffentlichungstag der Patentschrift: **17.10.84**

(51) Int. Cl.³: **A 61 B 5/04**

(21) Anmeldenummer: **79100299.1**

(22) Anmeldetag: **02.02.79**

(54) **Störungssicherer QRS-Detektor mit automatischer Schwellenwertbestimmung.**

(30) Priorität: **09.02.78 DE 2805482**

(43) Veröffentlichungstag der Anmeldung:
**22.08.79 Patentblatt 79/17**

(45) Bekanntmachung des Hinweises auf die
Patenterteilung:
**17.10.84 Patentblatt 84/42**

(84) Benannte Vertragsstaaten:
**CH FR GB NL SE**

(56) Entgegenhaltungen:
**DE-A-1 960 934**

**ELECTRONIC DESIGN (US), Band 17, nr.
17,16.August 1969,New York, A.K.GODDEN:
"Amplify biological signals with ICs", Seiten
218-224**

(73) Patentinhaber: **Hellige GmbH
Postfach 728 Heinrich-von-Stephan-Strasse 4
D-7800 Freiburg (DE)**

(72) Erfinder: **Hofmann, Gerhard H., Dipl.-Ing.
Friedrichstrasse 58
D-7800 Freiburg/Breisgau (DE)**

(74) Vertreter: **ter Meer, Nicolaus, Dipl.-Chem.Dr.
et al
Patentanwälte ter Meer, Müller, Steinmeister
Triftstrasse 4
D-8000 München 22 (DE)**

Courier Press, Leamington Spa, England.

## Beschreibung

Die Erfindung betrifft einen QRS-Detektor für gleichgerichtete QRS-Eingangs-Signale mit automatischer Schwellenwertbestimmung für Geräte zur Überwachung der Herztätigkeit, bei dem der Schwellenwert des Detektor-Einsatzpunkts in Abhängigkeit vom Eingangssignal durch eine dem als Komparator oder als Operationsverstärker mit mindestens zwei Eingängen ausgebildeten Detektor vorgeschaltete Einstellschaltung automatisch verschiebbar ist, die ein mit dem einen Eingang des Detektors verbundenes Tiefpaßfilter und einen vom gleichen Eingangssignal wie das Tiefpaßfilter beaufschlagten Spannungsteiler umfaßt, dessen Zwischenabgriff mit dem anderen Eingang des Detektors verbunden ist.

Ein QRS-Detektor dieser Art ist durch die DE—A 25 45 802 bekanntgeworden.

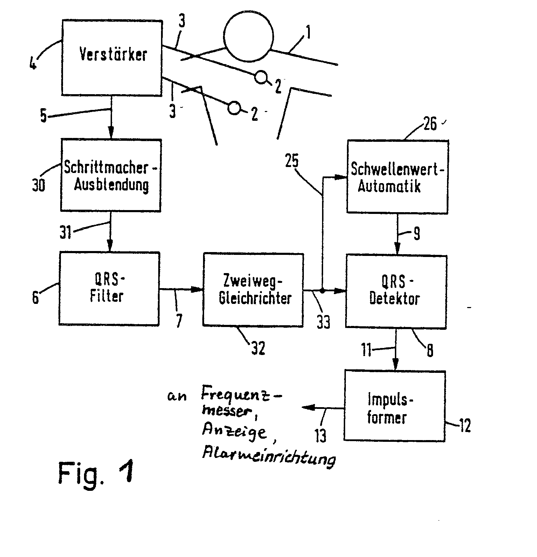

Die Funktionstüchtigkeit früherer Herzfrequenzmonitoren, wie Geräte der hier in Rede stehenden Art meist genannt werden, war durch eine zu geringe Selektivität der QRS-Filter eingeschränkt. Seit etwa 1970 sind Herzfrequenzmonitoren bekannt, die eine von der Polarität des QRS-Komplexes unabhängige Ansprechschwelle besitzen und außerdem zur Überwachung der Herztätigkeit von Patienten mit implantiertem oder externem Schrittmacher geeignet sind. Zur Verdeutlichung des allgemeinen Aufbaus solcher Herzfrequenzmonitoren wird auf das in Fig. 1 dargestellte Blockschaltbild eines Herzfrequenzmonitors mit Schwellenwert-Automatik für den QRS-Detektor und polaritätsunabhängiger Ansprechwelle hingewiesen, das nachstehend erläutert wird.

Die am Körper eines Patienten 1 durch im allgemeinen an der Oberfläche angebrachte Elektroden 2 abgenommene, meist EKG genannte Herzaktionsspannung gelangt über Elektrodenleitungen 3, einen Verstärker 4, eine Verbindungsleitung 5, eine zur Amplitudenselektion bestimmte, als "Schrittmacher-Ausblendung" 30 bezeichnete Schaltung sowie eine Verbindung 31 zu einem zur Selektion des QRS-Signals erforderlichen (linearen Frequenz-) Filter 6. Dem Filter 6 ist über eine Verbindung 7 ein Zweiweg-Gleichrichter 32 nachgeschaltet, der jegliche Vorzeichenabhängigkeit der QRS-Erkennung durch Bildung des Absolutbetrags der Signalspannung beseitigt.

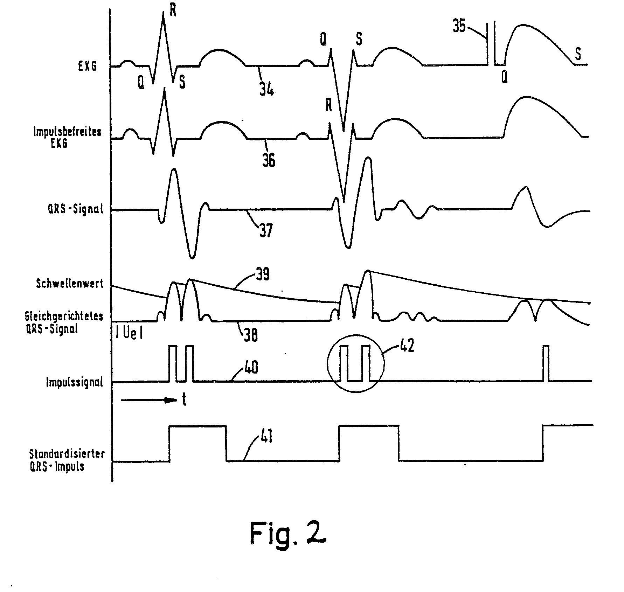

Zur Illustration der Funktion der Schrittmacher-Ausblendung 30 und des Zweiweg-Gleichrichters 32 wird auf die Darstellung in Fig. 2 hingewiesen. Durch Entfernung von Impulsen, z.B. Schrittmacherimpulsen 35, entsteht aus dem EKG 34 das impulsbefreite EKG 36, aus dem durch das Filter 6 ein QRS-Signal 37 und durch nachfolgende Betragsbildung im Zweiweg-Gleichrichter 32 ein gleichgerichtetes QRS-Signal 38 gebildet wird. Eine mit dem Gleichrichter durch eine Leitung 25 verbundene Schwellenwert-Automatik 26 (Fig. 1) erzeugt aus dem Signal 38 einen Schwellenwert 39, aus dem durch Verknüpfung mit dem Signal 38 durch einen QRS-Detektor 8 (Fig. 1), der dazu mit der Schwellenwert-Automatik durch die Leitung 9 und dem Zweiweg-Gleichrichter durch die Leitung 33 verbunden ist, ein Impulssignal 40 hergeleitet wird.

Aus Fig. 2 geht weiter hervor, daß zu jeder R-Zacke im EKG 34 nur ein standardisierter QRS-Impuls 41 in einem durch die Verbindung 11 an dem QRS-Detektor 8 angeschlossenen Impulsformer 12 (Fig. 1) erzeugt wird, und zwar auch dann, wenn zu einer R-Zacke im QRS-Detektor 8 eine an sich mehrdeutige Impulsgruppe 42 auftritt. Das wird durch die Eigenzeit des Impulsformers 12 erreicht. An diesen sind über eine Verbindung 13 ein Frequenzmesser, eine Anzeige- und eine Alarmeinrichtung angekoppelt, die bei physiologisch bedenklichem Herzrhythmus ein Warnsignal erzeugt.

Von großer Bedeutung für die Funktionssicherheit eines solchen Herzfrequenzmonitors sind die Eigenschaften der Schwellenwert-Automatik 26, besonders wenn es sich um die Überwachung eines EKG-Signals mit wechselnder Gestalt von QRS-Komplexen oder mit überlagerten Störspannungen handelt. Daraus erklärt sich der Bedarf für Ausführungsformen solcher Geräte mit Schwellenwert-Automatik und höherer Störspannungssicherheit.

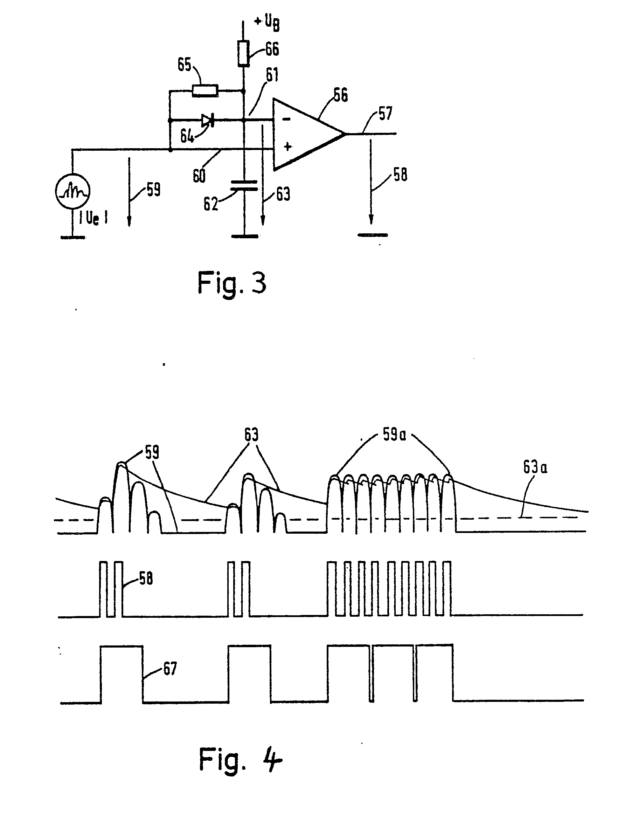

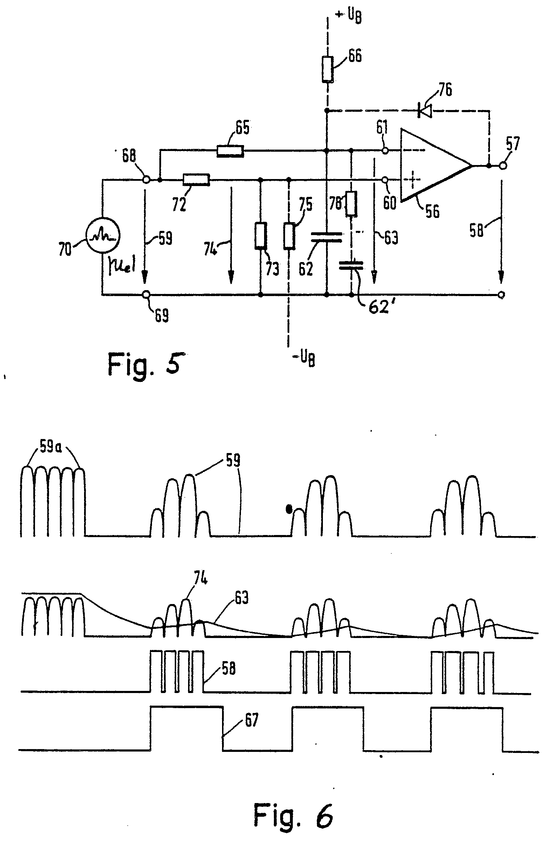

Die Fig. 3 zeigt einen QRS-Detektor mit automatischer Schwellenwertanpassung und einstellbarem Mindestschwellenwert, der sich auch zur QRS-Erkennung bei wechselnden QRS-Polaritäten eignet und an dem hinter dem QRS-Filter angeordneten Zweiweg-Gleichrichter 32 (Fig. 1) angeschlossen ist. Der Verstärker 56 dieses QRS-Detektors liefert an seinem Ausgang 57 ein positives Ausgangssignal 58, wenn das Signal 59 an seinem Eingang 60 größer wird als der an seinem anderen Eingang liegende, im Kondensator 62 gespeicherte Schwellenwert 63. Zugleich wird der Schwellenwert 63 erhöht, wenn die Eingangsspannung 59 die Spannung am Punkt 61 um mehr als die Flußspannung einer Diode 64 übersteigt. Die Wiederherstellung eines minimalen Schwellenwertes 63a (vgl. Fig. 4) erfolgt während der Aussteuerpausen durch Entladung des Kondensators 62 über den Widerstand 65 auf einen Endwert hin, der bestimmt ist durch die Betriebsspannung $+U_B$ und einen Spannungsteiler, bestehend aus dem Widerstand 65 und einem Widerstand 66.

In Fig. 4 sind die Signalspannungen dargestellt, die beim Betrieb des in Fig. 3 dargestellten QRS-Detektors mit Schwellenwert-Automatik auftreten. 59 bezeichnet das Eingangssignal, 63 den daraus gebildeten Schwellenwert, 58 das Impulssignal am Ausgang des QRS-Detektors und 67 das standardisierte QRS-Impulssignal, das aus 58 in dem Impulsformer 12 entsprechend Fig. 1 erzeugt wird.

Leider hat auch der in Fig. 3 dargestellte automatische QRS-Detektor nicht die Fähigkeit, QRS-komplexe im Signal 59 zu unterscheiden von Störwechselströmen 59a, wie durch den zeitlichen Verlauf der signal 58 und 67 in Fig. 4 zum Ausdruck kommt. Bei diesem QRS-Detektor können daher Störsignalwechselspannungen zu einer Fehlauslösung einer QRS-Anzeige führen.

Zur Verbesserung der Betriebseigenschaften eines Herzfrequenzmonitors ist es zweckmäßig, solche sich aus Störwechselspannungen insbesondere im Bereich der Netzfrequenz ergebende Fehltriggerungen des QRS-Detektors zu verhindern. Eine Anordnung dazu wird in der eingangs genannten DE—A 25 45 802 beschrieben; ihre Funktion beruht auf der Erzeugung eines Schwellenwertes durch Mittelwertbildung aus den Scheitelwerten (= Hüllkurve) des zugeführten, möglicherweise gefilterten, jedoch unter Umständen mit Störanteilen behafteten EKG-Signals und einer QRS-Erkennung immer dann, wenn der momentane Scheitelwert um einen bestimmten Betrag über dem gemittelten Scheitelwert liegt. Wird der Schwellenwert jedoch aus dem gemittelten Scheitelwert des EKG-Signals gewonnen, so ist bei schwankenden EKG- und damit QRS-Amplituden keine sichere Signalermittlung mehr möglich, da beispielsweise bei einem plötzlich auftretenden schwachen QRS-Signal der Schwellenwert zu hoch liegt.

Der Erfindung liegt daher die Aufgabe zugrunde, einen einfach aufgebauten, gegen periodische Störspannungen unempfindlichen QRS-Detektor mit automatischer Schwellenwertanpassung des Detektor-Einsatzpunkts der in der DE—A 25 45 802 beschriebenen Art zu schaffen, der auch bei schwachen EKG-Signalen noch sicher durch die QRS-Impulse getriggert wird.

Die Lösung dieser Aufgabe besteht nach der Erfindung darin, daß

— die dem Detektor vorgeschaltete Einstellschaltung eingangsseitig unmittelbar mit dem QRS-Signal als Nutzsignal, das ein durch Zweiweggleichrichtung erhaltenes Betragssignal ist, und der diesem Nutzsignal gegebenenfalls überlagerten Störkomponente als gemeinsamem Eingangssignal gespeist ist,

— einerseits das Tiefpaßfilter so bemessen ist, daß an seinem mit dem einen Eingang des Detektors verbundenen Ausgang der Schwellenwert als arithmetischer Mittelwert des Eingangssignals auftritt, und daß

— andererseits der Spannungsteiler so dimensioniert ist, daß ein aus dem Eingangssignal gebildetes und zu diesem im Verhältnis $1/k \leqq 2 : \pi$ stehendes zweites Signal an dem mit dem zweiten Eingang des Detektors verbundenen Zwischenabgriff auftritt, dessen Scheitelamplitude in Falle periodischer Störungen durch das gewählte Spannungsteilungsverhältnis von $1/k \leqq 2 : \pi$ kleiner ist als der zugehörige, über das Tiefpaßfilter am ersten Eingang des Detektors auftretende Schwellenwert.

Die Erfindung ist in Verbindung mit Untersuchungen an Herzfrequenzmonitoren entstanden; für solche Geräte stellt der Störungssichere QRS-Detektor gemäß der Erfindung eine wesentliche Verbesserung dar. Ein solcher QRS-Detektor eignet sich jedoch auch für andere Geräte im Bereich der medizinischen Technik zur Überwachung der Herztätigkeit von Lebewesen. Als Anwendungsbeispiele sie auf gesteuerte Defibrillatoren, Demand-Schrittmacher, auf Geräte zur synchronisierten Injektion, auf Geräte für synchrone, assistierte Zirkulation und andere Anwendungsmöglichkeiten hingewiesen, bei denen ein periodisch auftretendes, charakteristisches Signal schwankender Stärke zu erfassen ist, das leicht von periodischen Störspannungen überlagert sein kann.

Bei dem erfindungsgemäßen QRS-Detektor, dessen aktive Baugruppe aus einem Komparator- oder einem Operationsverstärker ·besteht, wird über die dem Detektor eingangsseitig vorgeschaltete Einstellschaltung — abgeleitet aus dem Eingangssignal — dem einen Detektoreingang, beispielsweise dem (—)Eingang eines Operationsverstärkers, der arithmetische Mittelwert des Eingangssignals als Schwellenwert aufgedrückt, während der andere Eingang, also beispielsweise der (+)Eingang des Operationsverstärkers, mit einem zum Eingangssignal im angegebenen Verhältnis stehenden zweiten Signal beaufschlagt wird.

Zur Erzeugung des Schwellenwerts als dem arithmetischen Mittelwert des Eingangssignals kann einfacher RC-Tiefpaß verwendet werden, der so dimensioniert ist, daß eine Integration über jeweils etwa eine Periode des QRS-Signals erfolgt. Um einen Mindestschwellwert festzulegen, kann der Schwellenwerteingang des Detektors über einen ohmschen Widerstand an ein Festpotential angebunden sein, wie dies oben in Verbindung mit Fig. 3 erläutert wurde. Dazu alternativ kann auch der andere, durch das zweite Signal beaufschlagte Detektoreingang auf ein tiefer liegendes Potential festgeklemmt werden.

Die Erfindung und vorteilhafte Einzelheiten werden nachfolgend anhand eines Ausführungsbeispiels unter Bezug auf die Zeichnung beschrieben.

Fig. 5 zeigt eine Ausführungsform eines erfindungsgemäßen QRS-Detektors mit Schaltungsergänzungsmöglichkeiten, die gestrichelt dargestellt sind;

Fig. 6 dient zur Erläuterung der Arbeitsweise der Grundausführung der Erfindung nach Fig. 5; und

Fig. 7 verdeutlicht die Arbeitsweise eines mit automatischer Schwellenwertbestimmung ausgerüsteten und auf definierte Mindestschwellenwerte festlegbaren QRS-Detektors nach der Erfindung.

Bei der in Fig. 5 dargestellten Ausführungsform eines störungssicheren QRS-Detektors 56 mit automatischer Schwellenwertbestimmung wird als Schwellenwert 63 der arithmetische Mittelwert des Eingangssignals benutzt. Damit jedoch, infolge von beispielsweise als Sinusbetrag auftretenden Störwechselspannungen 59a (Fig. 6) im Gegensatz zum Anschauungsbeispiel der Fig. 4 keine Impulse im Ausgangssignal 58 des QRS-Detektors auftreten, wird dem in diesem Fall als QRS-Detektor 56 benutzten Komparator als erstes Signal der Schwellenwert 63 und als zweites Signal ein Signal 74 zugeführt, das dem Eingangssignal 59 proportional ist und Scheitelamplituden aufweist, die im Falle eines Störsignals 59a kleiner sind als der zugehörige Schwellenwert 63. Dies wird in einfacher Weise durch geeignete Bemessung des von Widerständen 72 und 73 gebildeten Spannungsteilers erreicht. Diese Maßnahme beeinträchtigt nicht die Funktionstüchtigkeit des QRS-Detektors, wie noch dargestellt werden wird.

Zur Bemessung des Spannungsteilers 72, 73 wird zurückgegriffen auf einen aus der Theorie der Gleichrichter bekannten Zusammenhang zwischen Scheitelwert û und arithmethischem Mittelwert ū bei einem durch Zweiweggleichrichtung aus einer Sinuswechselspannung erzeugten Signal:

$$\hat{u} : \overline{u} = \pi : 2 = 1{,}57 : 1$$

Um diese Gleichungsbeziehung zu erfüllen, wird beim QRS-Detektor nach Fig. 5 das von der durch das Symbol 70 dargestellten Quelle gelieferte Eingangssignal 59, welches ein durch Zweiweggleichrichtung erhaltenes Betragssignal ist, dem durch die Klemmen 68 und 69 dargestellten Eingang zugeführt. Die Verbindung zwischen Klemme 68 und der Eingangsklemme 61 des als QRS-Detektor benutzten Komparators 56 erfolgt durch den Widerstand 65. Ergänzend dazu liegt ein Kondensator 62 zwischen den Klemmen 61 und 69, der mit dem Widerstand 65 einen RC-Tiefpaß zur Erzeugung des Schwellenwerts 63 als arithmetischer Mittelwert des Eingangssignals 59 bildet.

Die Verbindung zwischen Klemme 68 und Eingangsklemme 60 des Komparators 56 erfolgt über den Widerstand 72; der weitere Widerstand 73 ist zwischen den Klemmen 60 und 69 engeordnet. Mit dem von diesen Widerständen 72 und 73 gebildeten Spannungsteiler wird das Verhältnis zwischen der Amplitude des Eingangssignals 59 und der Amplitude des Signals 74 zwischen den Klemmen 60 und 69 auf einen Wert k ≧ 1,57 eingestellt.

Ein solcher QRS-Detektor liefert also nur dann am Ausgang 57 des Detektors 56 Impulse, wenn der mit

$$\frac{1}{k}$$

multiplizierte Scheitelwert des Eingangssignals 59 größer ist als der arithmetische Mittelwert des Eingangssignals 59. Diese Bedingung ist erfüllt bei Verarbeitung echter EKG-Signale, nicht jedoch bei periodischen Störwechselspannungen, d.h., die Schaltung hat eine Entstörwirkung. störwirkung.

Zur Ergänzung dieser Entstörwirkung kann der beschriebene QRS-Detektor mit einer Beschaltung zur Vorgabe einer Mindestschwelle und einer Automatik zum Aufbau eines amplitudenabhängigen Schwellenwerts kombiniert werden. Zur Realisierung der Mindestschwelle wird entweder mit einem *Widerstand 66 zwischen dem Eingang 61 und der Betriebsspannung +U_B der Wert der Spannung über dem Kondensator 62 erhöht, oder es wird mit einem Zusätzlichen Widerstand 75 zwischen einer Betriebsspannung —U_B und dem Eingang 60 die Spannung an diesem Eingang 60 erniedrigt.

Der Aufbau des amplitudenabhängigen Schwellenwerts erfolgt durch Einspreicherung des Scheitelwerts der Spannung des Signals 74 in den Kondensator 62. Dazu kann bei der Schaltung nach Fig. 5 der Detektor 56 als Differenzverstärker mit einem Plus-Eingang 60, einem Minus-Eingang 61 und einem Ausgang 57 ausgeführt werden, ergänzt um eine Diode 76, deren Anode mit dem Verstärkerausgang 57 und deren Kathode mit dem Verstärkereingang 61 verbunden ist.

Zur Erhöhung der Funktionstüchtigkeit bei schwankenden QRS-Amplituden kann man bei einem QRS-Detektor nach Fig. 5 mit Unterdrückung von Störwechselspannungen und einem abhängig von der erkannten QRS-Amplitude erhöhten Mindestschwellenwert den Kondensator 62 durch eine Serienschaltung aus einem Kondensator 62' und einem Widerstand 78 ersetzen. Man erreicht dadurch, daß über die Diode 76 in den Kondensator 62' ein Schwellenwert eingespeichert wird, der unterhalb des Scheitelwerts der Spannung des Signals 74 bleibt. An der Wechselstromentstörung durch Bildung eines Schwellenwerts aus dem Signalmittelwert tritt dadurch keine Änderung ein.

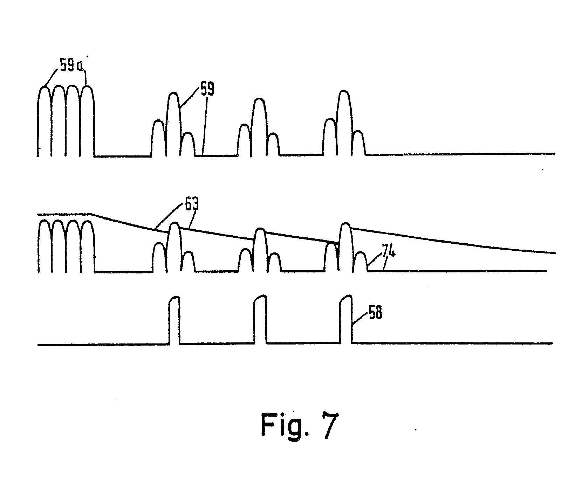

Die Diagramme in Fig. 7 stellen die Funktionsweise eines störsicheren QRS-Detektors mit automatischer Schwellenwertbestimmung dar, der in der in Fig. 5 dargestellten Weise aus den Elementen 56, 65, 72, 73, 76, 62', 78 sowie 66 oder 75 zusammengesetzt ist.

Die Schaltung gemäß Fig. 5 kann unschwer für Eingangssignal 59 mit umgekehrter Polarität, d.h. mit gegen Anschluß 69 negativen Auslenkungen modifiziert werden. Dazu muß die Diode 76 umgekehrt eingesetzt werden, und der für die Minimalschwelle verantwortliche Widerstand 66 bzw. 75 muß an der entgegengesetzten Betriebsspannung —U_B bzw. +U_B angeschlossen werden. In diesem Fall treten alle in Fig. 7 dargestellten Signale, also 59, 74, 63 und 58, mit umgekehrter Polarität auf.

* bereits von Fig. 3 bekannten

**Patentansprüche**

1. QRS-Detektor für gleichgerichtete QRS-Eingangs-Signale (59) mit automatischer Schwellenwertbestimmung für Geräte zur Überwachung der Herztätigkeit, bei dem der Schwellenwert des Detektor-Einsatzpunkts in Abhängigkeit vom Eingangssignal (59) durch eine dem als Komparator oder als Operationsverstärker mit mindestens zwei Eingängen (60, 61) ausgebildeten Detektor (56) vorgeschaltete Einstellschaltung automatisch verschiebbar ist, die ein mit dem einen Eingang (61) des Detektors (56) verbundenes Tiefpaßfilter (65, 62) und einen vom gleichen Eingangssignal wie das Tiefpaßfilter (62, 65; 62', 65) beaufschlagten Spannungsteiler (72, 73) umfaßt, dessen Zwischenabgriff mit dem anderen Eingang (60) des Detektors (56) verbunden ist, dadurch gekennzeichnet, daß dadurch gekennzeichnet, daß

— die dem Detektor (56) vorgeschaltete Einstellschaltung (62, 65, 72, 73; 62', 65, 72, 73) eingangsseitig unmittelbar mit dem QRS-Signal als Nutzsignal, das ein durch Zweiweggleichrichtung erhaltenes Betragssignal ist, und der diesem Nutzsignal gegebenenfalls überlagerten Störkomponente als gemeinsamem Eingangssignal (59) gespeist ist,

— einerseits das Tiefpaßfilter (62, 65; 62', 65) so bemessen ist, daß an seinem mit dem einen Eingang (61) des Detektors (56) verbundenen Ausgang der Schwellenwert (63) als arithmetischer Mittelwert des Einganssignals (59) auftritt, und daß

— andererseits der Spannungsteiler (72, 73) so dimensioniert ist, daß ein aus dem Eingangssignal (59) gebildetes und zu diesem im Verhältnis $1/k \leq 2:\pi$ stehendes zweites Signal (74) an dem mit dem zweiten Eingang (60) des Detektors (56) verbundenen Zwischenabgriff auftritt, dessen Scheitelamplitude im Falle periodischer Störungen durch das gewählte Spannungsteilungsverhältnis von $1/k \leq 2:\pi$ kleiner ist, als der zugehörige, über das Tiefpaßfilter (62, 65; 62', 65) am ersten Eingang (61) des Detektors (56) auftretende Schwellenwert.

2. QRS-Detektor nach Anspruch 1, dadurch gekennzeichnet, daß die Einstellschaltung (62, 65, 72, 73) einen Widerstand (66 oder 75) zur Vorgabe einer Mindestschwelle für den Schwellenwert des Detektor-Einsatzpunktes aufweist. aufweist.

3. QRS-Detektor nach Anspruch 1 oder 2, dadurch gekennzeichnet, daß die Einstellschaltung (62, 65, 72, 73; 62', 65, 72, 73) zur automatischen Amplitudenanpassung des Schwellenwerts in Abhängigkeit von den Amplituden des proportional zum Eingangssignal untersetzten zweiten Signals (74) eine Rückkoppelung vom Detektorausgang (57) auf den mit dem Schwellenwert beaufschlagten Eingang (61) des Detektors (56) aufweist.

4. QRS-Detektor nach Anspruch 3, dadurch gekennzeichnet, daß der Detektor (56) ein als Differenzverstärker geschalteter Operationsverstärker ist und die Rückkoppelung durch eine vom Detektorausgang (57) zum Schwellenwert-Eingang (61) in Durchlaßrichtung gepolte Diode (76) gebildet ist, über die der Schwellenwert auf den jeweils auftretenden Scheitelwert des zweiten Signals hochgezogen wird.

5. QRS-Detektor nach Anspruch 4, dadurch gekennzeichnet, daß zwischen dem Schwellenwert-Eingang (61) und dem Kondensator (62') des als RC-Glied ausgebildeten Tiefpaßfilters (62', 65) ein ohmscher Widerstand (78) geschaltet ist, der sicherstellt, daß die Schwellenspannung immer unterhalb des Spitzenwerts des QRS-Signals bleibt.

**Revendications**

1. Détecteur QRS pour des signaux d'entrée QRS redressés (59) avec détermination automatique de valeur de seuil pour des appareils de contrôle de l'activité cardiaque, dans lequel la valeur de seuil du point de fonctionnement du détecteur est modifiable en fonction du signal d'entrée (59) par un circuit de réglage disposé en amont du détecteur (56) et agencé en comparateur ou en amplificateur opérationnel avec au moins deux entrées (60, 61) ledit circuit comprenant un filtre passe-bas (65, 62) relié à une première entrée (61) du détecteur (56) et un diviseur de tension (72, 73) recevant le même signal d'entrée que le filtre passe-bas (62, 75; 62', 65) et dont une borne intermédiaire est reliée à l'autre entrée (60) du détecteur (56), caractérisé par le fait

— que le circuit de réglage (62, 65, 72, 73; 62', 65, 72, 73) en amont du détecteur (56) reçoit comme signal d'entrée commun (59) du côté d'entrée directement le signal QRS en tant que signal utile qui est un signal de somme, obtenu par redressement à double voie, et la composante de perturbation le cas échéant superposée à ce signal utile,

— que d'une part le filtre passe-bande (62, 65; 62', 65) est dimensionné de telle manière que la valeur de seuil est appliquée à sa sortie reliée à l'entrée (61) détecteur (56) comme moyenne arithmétique du signal d'entrée (59) et

— que d'autre part le diviseur de tension (72, 73) est dimensionné de telle manière qu'à la borne intermédiaire reliée à la seconde entrée (60) du détecteur (56) etst appliqué un deuxième signal (74) formé à partir du signal d'entrée (59), dans la proportion de $1/k \leq 2:\pi$ par rapport à celui-ci et dont l'amplitude de crête dans le cas de perturbations périodiques est plus faible dans le rapport de division de tension choisi de $1/k \leq 2:\pi$ que la valeur de seuil correspondante appliquée à travers le filtre passe-bande (62, 65; 62', 65) à la première entrée (61) du détecteur (56).

2. Détecteur QRS selon la revendication 1, caractérisé par le fait que le circuit de réglage (62, 65, 72, 73) présente un résistance (66 ou 75) pour fournir un seuil minimal pour la valeur de seuil du point de fonctionnement du détecteur.

3. Détecteur QRS selon la revendication 1 ou 2, caractérisé par le fait que le circuit de réglage (62, 65, 72, 73; 62', 65, 72, 73) pour l'adaptation d'amplitude automatique de la valeur de seuil en fonction des amplitudes du deuxième signal (74), réduit proportionnellement au signal d'entrée, présente un couplage de réaction de la sortie du détecteur (57) à l'entrée (61) détecteur (56) à laquelle est appliquée la valeur de seuil.

4. Détecteur QRS selon la revendication 3, caractérisé par le fait que le détecteur (56) est un amplificateur opérationnel agencé en amplificateur différenciateur et que le couplage de réaction est formé d'une diode (76) polarisée dans le sens de conduction de la sortie du détecteur (57) vers l'entrée de valeur de seuil (61), diode par laquelle la valeur de seuil est à chaque fois élevée jusqu'au seuil de crête du second signal.

5. Détecteur QRS selon la revendication 4, caractérisé par le fait qu'entre l'entrée de valeur de seuil (61) et le condensateur (62') du filtre passe-bande (62', 65) réalisé sous la forme d'un élément RC est disposée une résistance ohmique (78) qui assure que la tension de seuil reste toujours en dessous de la valeur de pointe du signal QRS.

**Claims**

1. QRS detector for rectified QRS input signals (59), including automatic threshold value determination for devices for monitoring heart activity, in which detector the threshold value of the detector turn-on point can be automatically displaced as a function of the input signal (59) by an adjustment circuit which precedes the detector (56) which is constructed as a comparator or as an operational amplifier having at least two inputs (60, 61), which adjustments circuit comprises a low-pass filter (65, 62) which is connected to the one input (61) of the detector (56) is fed directly with the QRS signal which the same input signal is applied as the low-pass filter (62, 65; 62', 65), the intermediate tap of which divider in connected to the other input (60) of the detector (56), characterised in that

— the input of the adjustment circuit (62, 65, 72, 73; 62', 65, 72, 73) preceding the detectpr (56) is fed directly with the QRS signal as a useful signal, which is a quantity signal obtained by two-way rectification, and with the noise component which is possibly superimposed on this useful signal as a joint input signal (59),

— on the one hand, the low-pass filter (62, 65; 62', 65) is dimensioned in such a manner that at its output connected to the one input (61) of the detector (56) the threshold value (63) occurs as the arithmetic mean of the input signal (59), and that

— on the other hand, the voltage divider (72, 73) is dimensioned in such a manner that a second signal (74) formed from the input signal (59) and having a relationship to the latter of $1/k \leq 2 : \pi$ occurs at the intermediate tap connected to the second input (60) of the detector (56), the peak amplitude of which second signal is less, in the case of periodic disturbances, as a result of the voltage dividing ratio of $1/k \leq 2 : \pi$ selected, than the associated threshold value occurring via the low-pass filter (62, 65; 62', 65) at the first input (61) of the detector (56).

2. A QRS detector according to Claim 1, characterised in that the adjustment circuit (62, 65, 72, 73) is provided with a resistance (66 or 75) for presetting a minimum threshold value for the threshold value of the detector turn-on point.

3. A QRS detector according to Claim 1 or 2, characterised in that the adjustment circuit (62, 65, 72, 73; 62', 65, 72, 73) for the automatic amplitude matching of the threshold value as a function of the amplitudes of the second signal (74) which is stepped down proportionally to the input signal, is provided with a feedback from the detector output (57) to the input (61) of the detector (56), to which the threshold value is applied.

4. A QRS detector according to Claim 3, characterised in that the detector (56) is an operational amplifier which is connected as a differential amplifier and that the feedback is formed by a diode (76) connected in the conducting direction from the detector outut (57) to the threshold value input (61), via which diode the threshold value is pulled up to the peak value, accurring, in each case, of the second signal.

5. A QRS detector according to Claim 4, characterised in that between the threshold value input (61) and the capacitor (62') of the low-pass filter (62, 65) constructed as an RC section, an ohmic resistance (78) is connected which ensures that the threshold voltage always remains below the peak value of the QRS signal.

Fig. 1

# 0·003·567

**Fig. 2**

Fig. 3

Fig. 4

0 003 567

Fig. 5

Fig. 6

4

Fig. 7